# EUROPEAN PATENT APPLICATION

(11) **EP 4 714 332 A2**
(43) Date of publication of application: **25.03.2026**
(21) Application number: 26150391.6
(22) Date of filing: 13.01.2022
(51) Int. Cl.: A61B 1/00

(54) **COUPLING DEVICE FOR AN ENDOSCOPE WITH AN ADJUSTABLE OPTICAL LENS**

(30) Priority: 14.01.2021 US 202163137691 P
(62) Divisional of application: 22740029.8
(71) Applicant: GI Scientific, LLC, Arlington, VA 22203 (US)
(72) Inventor: MILLER, Scott, Arlington, 22207 (US)
(74) Representative: Richardt Patentanwälte PartG mbB

(57) **Abstract**

A coupler device is provided for covering and at least partially sealing a portion of the working end of an endoscope, and an actuator for providing energy to the endoscope to automatically and/or manually adjust the focal length of the optical lens. The coupler device protects the scope and its components to reduce the ingress of debris, fluid and other matter, thereby reducing infection risk. In addition, the coupler device provides a mechanism to provide adjustable focus and more viewing power from the lens, thereby minimizing manual movement of the endoscope during operation and allowing for the design of more compact and maneuverable endoscopes.

## Description

### CROSS-REFERENCE TO RELATED APPLICATIONS

This application claims the benefit of U.S. Provisional Application No.63/137,691 , filed January 14, 2021, the entire contents of which are incorporated herein by reference for all purposes.

### BACKGROUND

Recent advances in optical imaging technology have allowed many medical procedures to be performed today in a minimally invasive manner. The evolution of the more sophisticated, flexible scope with advanced visual capabilities has allowed access to regions deep within the human body that could only be achieved before with invasive surgical intervention. This modern day convenience has resulted in an increase in the demand for, as well as the number of, endoscopic, laparoscopic, arthroscopic, ophthalmoscopic, or other remote imaging visualization procedures performed every year in the U.S and globally. While these procedures are relatively safe, they are not without risks.

Endoscopy, for instance, is a procedure in which a lighted visualization device called an endoscope is inserted into the patient's body to look inside a body cavity, lumen, organ or in combination, for the purpose of examination, diagnosis or treatment. The endoscope may be inserted through a small incision or through a natural opening of the patient. In a bronchoscopy, the endoscope is inserted through the mouth, while in a sigmoidoscopy, the endoscope is inserted through the rectum. Unlike most other medical imaging devices, endoscopes are inserted directly into the organ, body cavity or lumen.

Today, most endoscopes are reused. This means that, after an endoscopy, the endoscope goes through a cleaning, disinfecting or sterilizing, and reprocessing procedure to be introduced back into the field for use in another endoscopy on another patient. In some cases, the endoscope is reused several times a day on several different patients.

While the cleaning, disinfecting and reprocessing procedure is a rigorous one, there is no guarantee that the endoscopes will be absolutely free and clear of any form of contamination. Modern day endoscopes have sophisticated and complex optical visualization components inside very small and flexible tubular bodies, features that enable these scopes to be as effective as they are in diagnosing or treating patients. However, the tradeoff for these amenities is that they are difficult to clean because of their small size, and numerous components. These scopes are introduced deep into areas of the body which expose the surfaces of these scopes to elements that could become trapped within the scope or adhere to the surface, such as body fluids, blood, and even tissue, increasing the risk of infection with each repeated use.

Endoscopes used in the gastrointestinal tract, such as endoscopic ultrasound scopes (EUS) and duodenoscopes with side-viewing capability, have an added complexity in that they are in a bacteria rich environment. Typical duodenoscopes and EUS scopes have internal moving components like an elevator with hinges attached to a cable for actuation. The elevator is used to deflect and therefore change the direction of instruments passed down the scope's working channel. This elevator is beneficial in that it can allow the user to change the direction of a wire or a catheter to direct the wire or catheter into a specific opening, so that one or more instruments can be turned to enter a particular body lumen or to penetrate or sample tissue. However, given the size, location and movement of the elevator during use, the elevator creates cleaning issues, including the risk that bacteria finds its way into the elevator's hinges and other hard to clean locations on the scope. This provides an opportunity for bacteria to colonize and become drug resistant, creating the risk of significant illness and even death for a patient. This infection risk is also present in the cable mechanisms that are used to move the elevator mechanism back and forth and in other aspects of current scope designs. Moreover, in addition to the health risks posed by bacterial contamination, the accumulation of fluid, debris, bacteria, particulates, and other unwanted matter in these hard to clean areas of the scope also impact performance, shortening the useful life of these reusable scopes.

Another drawback with existing endoscopes is that conventional lens focusing systems are not suitable due to restrictions in diameter and lens displacement range. Endoscopes typically contain a series of fixed lenses along an optical path to provide an image of an object at one end of the endoscope to a user viewing thorough the other end. Each of these lenses has a fixed shape or focal length. Thus, the use of conventional lenses within endoscopes defines a specific working distance at which the object being viewed is in focus. Deviating away from this working distance will cause the object to appear blurry to the user viewing it at the opposite end. Thus, the endoscope must be kept stationary at a certain distance away from an object in order to maintain clear focus on the object. Changing the working distance, or focal length, can be achieved by switching between lenses of various optical powers within the endoscope. However, once the endoscope is in use, it is difficult to change any of the lenses used within it. Moreover, only discrete working distances and magnification powers may be set using stationary lenses with rigid shapes. Accordingly, the endoscope operator needs to move and refocus the endoscope more frequently, increasing the endoscopy procedure time and potentially increasing patient discomfort during certain procedures.

Accordingly, it is desirable to provide devices which serve as convenient accessories for currently existing endoscopes to reduce the risk of contamination and infection, while also improving the performance of the endoscope. It is particularly desirable to provide an accessory or companion device for endoscopes having the capability of adjusting the focal length of an optical lens of the endoscope to minimize manual movement of the endoscope during the procedure and to allow for the design of more compact and maneuverable endoscopes that are also more easily cleaned and sterilized.

### SUMMARY

A coupler device is provided for covering and at least partially sealing a portion of the working end of an endoscope, and an actuator for providing energy to the endoscope to automatically and/or manually adjust the focal length of the optical lens. The coupler device protects the scope and its components to reduce the ingress of debris, fluid and other matter, thereby reducing infection risk. In addition, the coupler device provides a mechanism to provide adjustable focus and more viewing power from the lens (i.e., zooming capability), thereby minimizing manual movement of the endoscope during operation and allowing for the design of more compact and maneuverable endoscopes.

In one aspect, a coupler device for use with an endoscope having an optical lens includes a main body comprising a visualization section for allowing viewing of tissue by the endoscope and a proximal end configured for removable attachment to a distal end portion of the endoscope. The coupler device further includes an actuator positioned to be in operational proximity to the optical lens when the proximal end of the main body is attached to the distal end portion of the endoscope. The actuator is configured to alter a curvature or shape of the optical lens, thereby adjusting the focal length of the lens.

The coupler device allows the operator to adjust the optical lens to obtain additional positive power correction as needed to view objects at further distances (i.e., focus up close or zoom in on objects without moving the endoscope). Alternatively, the power may be corrected automatically as part of a feedback control loop. In addition the coupler device allows the optical lens to be adjusted continuously over a desired power range. For example, the focal length of the lens may be adjusted to substantially match the distance between a distal end of the endoscope and a desired object, allowing the operator to move the endoscope closer or further away from the object while maintaining focus.

The coupler device also allows for the design of endoscopes having fewer optical lenses along the optical path between the viewer and the target site than conventional endoscopes. The ability to adjust at least one of the optical lenses reduces the need for multiple fixed lenses to change the optical power of the endoscope. This allows for the design of even more compact and maneuverable endoscopes, thereby increasing patient comfort and potentially providing access to difficult target areas within the patient that are difficult to reach with conventional scopes.

In certain embodiments, the actuator may include, for example, a controller, a power supply, such as a battery or other suitable source of power, and one or more coupling elements for coupling either directly to the optical lens or indirectly through another element within the endoscope. The power supply provides energy to the optical lens through the coupling elements in order to adjust the surface profile or curvature of the lens, thereby adjusting the focus of the lens.

In certain embodiments, the power supply, the coupling elements or another connector within the actuator may be configured to automatically adjust the curvature of the lens to refocus the lens on an object as the endoscope is moved within the patient. In these embodiments, the actuator may include an autofocus light sensor or similar device and a controller that evaluates signals from the autofocus light sensor. The controller then automatically supplies energy to the lens, as necessary, to adjust the focal length of the lens until the image is in focus.

In other embodiments, the actuator may include a controller that allows the operator to manually adjust the actuator and the focal length of the optical lens when desired. The controller may be controlled from a user interface device that includes one or more input controls for sending electrical signals to the actuator in the coupler device. The user interface device may be incorporated into the handle of the endoscope or on a separate device. The electrical signals may be transmitted through a connector that extends through the endoscope to the coupler device or the actuator may be controlled wirelessly through a signal that is delivered externally from the patient to the coupler device.

In other embodiments, the power supply may be located externally to the coupler device and/or the patient and the power or energy for adjusting the optical lens is delivered wirelessly to the actuator in the coupler device (or through a connector in the endoscope). In one such embodiment, the actuator comprises a receiver configured to wirelessly receive energy from a power source and to transmit the energy to the optical lens. Alternatively or in addition, the receiver may be configured to receive electrical signals (e.g., command inputs) from a remote source to allow the operator to control the actuator. The receiver may be disposed within the main body or on the outer surface of the coupler device. The source of energy and/or the controller may be located remotely from the coupler device, such as exterior to the patient. The energy may comprise an electromagnetic field, an electromagnetic radiation or any other suitable energy that may be transmitted wirelessly from the source of energy to the actuator.

In certain embodiments, the energy comprises an electromagnetic field and the energy is transmitted through inductive coupling or capacitive coupling. In the former embodiment, the source of energy comprise a first magnetic coil or coils and the actuator comprises a second magnetic coil or coils. In the latter embodiment, the source of energy includes a first electrode or electrode(s) and the actuator includes a second electrode or electrodes(s). The first and second electrodes are configured to transmit energy through capacitive coupling from the first electrode(s) to the second electrode(s). In either embodiment, the actuator may further comprise a rectifier or other suitable mechanism for converting the electromagnetic field to a DC or AC electric current, or to another form of energy suitable for adjusting a characteristic of the lens.

In another embodiment, the energy comprises electromagnetic radiation, such as microwaves, laser beams or the like. In these embodiments, the source of power comprises one or more antennas or other suitable coupling devices and the actuator comprises one or more receivers. The electromagnetic energy is transmitted wirelessly from the antenna(s) to the receiver(s). The actuator may further comprise a rectifier or other suitable mechanism for converting the electromagnetic radiation to a suitable energy form for adjusting a characteristic of the lens.

The actuator further comprises one or more coupling elements that are positioned to be in operational proximity to the optical lens when the proximal end of the main body of the coupler device is attached to the distal end of the scope. The coupling elements may be in direct contact with the optical lens (or another element of the scope that is in contact with the optical lens) or they may be positioned in close enough proximity to the optical lens to transmit the energy thereto.

In certain embodiments, the optical lens comprises one or more adjustable lenses and, in some embodiments, one or more rigid or fixed lenses. The adjustable lens(es) preferably comprise fluid lenses that have one or more fluids enclosed within a chamber having substantially clear proximal and distal surfaces to allow light to pass through the fluid(s) within the chamber. The focal length of the fluid lens may be adjusted by changing the shape or the index distribution of the fluid. In certain embodiments, the fluid lens is configured such that the surface profile or radius of curvature of the fluid(s) determines the focal length of the optical lens. By altering the surface profile or radius of curvature of the fluid(s), the focal length of the lens can be adjusted. The surface profile of the fluid(s) may be adjusted by the application of energy directly to, or through, the fluid(s), or indirectly to another mechanism that adjusts the pressure, volume or other characteristics of the fluid.

In one embodiment, the actuator comprises one or more electrodes in operational proximity to the fluid lens when the proximal end of the coupler device is attached to the distal end of the endoscope. The actuator is configured to apply a voltage across the electrodes to change the shape or curvature of the fluid(s) therein. In an exemplary embodiment, the chamber of the fluid lens houses at least two different, immiscible liquids, such as water and oil, or other suitable liquids. The voltage changes the shape of the water within the fluid lens to alter the curvature of the lens and its focal length.

In another embodiment, the lens chamber includes a curved portion that functions as the lens and a reservoir portion fluidly coupled to the curved portion. The chamber is filled with a single fluid, such as water, oil, air, or any suitable fluid. The lens may further include an outer membrane that encloses and seals the chamber. In this embodiment, the actuator is configured to exert a pressure on the membrane to move the fluid from the reservoir to the curved portion of the lens. Alternatively, the actuator may be configured to exert pressure directly on the fluid reservoir to force the fluid from the reservoir into the curved portion of the lens. The actuator may be driven by a variety of energy sources known to the art, such as electric current, magnetic energy, mechanical energy, acoustic energy and the like. As the membrane exerts more pressure on the chamber, the fluid is driven from the curved portion to the reservoir, thereby decreasing the curvature of the lens. Removing this pressure causes the fluid to displace into the curved portion, thereby increasing the curvature of the lens.

In some embodiments, the coupler device allows the user to articulate the working channel of the device in the direction preferred by the user of the endoscope, so that a wire, catheter or other instrument being advanced down the working channel of the endoscope can direct the wire or catheter or other instrument in a preferred direction different than the angle at which the instrument would exit the endoscope if the coupler device was not in place or if an elevator in the scope is not used. This redirection of an instrument has the benefit of assisting with the navigation of the device, while not allowing fluid, debris, particulate matter, bacteria and other unwanted elements to enter hard to clean areas of the endoscope, especially at the distal end of the endoscope.

In other embodiments, the coupler device includes a flexible working channel extension that extends the working or biopsy channel of the scope and can be angularly adjustable. The flexible working channel extension may be adjustable by an elevator or cable passing through the endoscope. Alternatively, the coupler device may include its own actuator, such as an elevator, cable, or similar actuation means, for adjusting the working channel extension and thereby articulating instruments passing through the endoscope. The actuator may be powered by any suitable source of energy, such as a motor or the like. The source of energy may be coupled to the actuator either directly through the scope, or indirectly through magnetic, electric, or some other source of wireless energy. The source of energy may be disposed within the coupler device, or it may be external to the coupler device (i.e., either disposed on the proximal end of the scope or external to the patient).

In some embodiments, the actuator of coupler device provides energy to both the articulating mechanism and the optical lens in the scope. In this manner, providing energy to the coupler device allows for articulation of an instrument passing through a working channel in the scope and adjustment of the focal length of the optical lens.

In some embodiments, the coupler device may be formed of an optically clear material that covers the end of the endoscope and seals the end of the endoscope, allowing visualization of the endoscope's camera without obscuring the view by the device. The optically clear material may also cover the endoscope's light guide to allow the light projected by the endoscope to illuminate the field of view of the endoscope. In some embodiments, the optically clear material may include navigation markers to orient the user when visualizing tissue, such as markers to identify the relative position of the scope as the user visualizes the tissue through the optically clear material.

In some embodiments, the coupler device may be integrated into a scope and configured to be detachable and reusable for separate cleaning, including manual cleaning, in an autoclave, an ETO sterilizer, gamma sterilizer, and other sterilization methods.

In some embodiments, the coupler device may cover the entire distal end of the endoscope, or may just cover hard to clean areas. In some embodiments, the coupler device may cover the distal end of the endoscope, or a portion thereof, or it may include a sheath attached to the coupler device which covers the entirety of the scope that is exposed to fluid, debris, particulate matter, bacteria and other unwanted elements.

It is to be understood that both the foregoing general description and the following detailed description are exemplary and explanatory only and are not restrictive of the description. Additional features will be set forth in part in the description which follows or may be learned by practice of the devices and methods described herein.

### BRIEF DESCRIPTION OF THE DRAWINGS

The accompanying drawings, which are incorporated in and constitute a part of this specification, illustrate several embodiments and together with the description, serve to explain the principles herein.
FIG. 1 is a partial cross-sectional view of the proximal portion of a representative endoscope;
FIG. 2 is a perspective view of the distal end portion of a representative side-viewing endoscope;
FIGS. 3A and 3B are cross-sectional views of an adjustable optical lens for use with an endoscope;
FIGS. 4A and 4B are perspective views of another embodiment of an adjustable optical lens for use with an endoscope;
FIGS. 5A and 5B are isometric views of an exemplary embodiment of a coupler device in use with a side-viewing scope;
FIGS. 6A and 6B show partial cutaway views of the coupler device of FIGS. 5A and 5B, respectively, in use with a representative endoscope;
FIG. 7 shows another cutaway view of the coupler device of FIGS. 5A and 5B in use with a representative endoscope;
FIG. 8 shows still another cutaway view of the coupler device of FIGS. 5A and 5B in use with a representative endoscope;
FIG. 9 is a schematic view of a wireless energy delivery system;
FIG. 10 is a schematic view of another embodiment of a wireless energy delivery system
FIG. 11A illustrates different components of a system for controlling aspects of a coupler device;
FIG. 11B is a schematic view of a control system for controlling an actuator within an optical coupler'
FIG. 12A is a cutaway side view of the coupler device of FIGS. 5A and 5B in a first position;
FIG. 12B is a cutaway side view of the coupler device of FIGS. 5A and 5B in a second position;
FIG. 13 is a cutaway side view of the coupler device of FIGS. 5A and 5B in a third position;
FIG. 14 is an enlarged side view of a membrane of the coupler device of FIGS. 5A and 5B;
FIG. 15 is a side view of an alternative embodiment of an optical coupler;
FIG. 16 is a cross-sectional view of the optical coupler of FIG. 15;
FIG. 17 is a cross-sectional view of the optical coupler of FIG. 15, the optical coupler being attached to a representative endoscope;
FIG. 18 is a side view of the distal end portion of a representative end viewing endoscope;
FIGS. 19A and 19B are isometric views of an exemplary embodiment of the coupler device in use with an end viewing scope;
FIGS. 20A and 20B show partial cutaway views of the coupler device of FIGS. 19A and 19B, respectively;
FIG. 21 is a cutaway side view of the coupler device in a first position;
FIG. 22 is a cutaway side view of the coupler device in a second position; and
FIG. 23 is a cutaway side view of the coupler device in a third position;

### DETAILED DESCRIPTION

This description and the accompanying drawings illustrate exemplary embodiments and should not be taken as limiting, with the claims defining the scope of the present description, including equivalents. Various mechanical, compositional, structural, and operational changes may be made without departing from the scope of this description and the claims, including equivalents. In some instances, well-known structures and techniques have not been shown or described in detail so as not to obscure the description. Like numbers in two or more figures represent the same or similar elements. Furthermore, elements and their associated aspects that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment. Moreover, the depictions herein are for illustrative purposes only and do not necessarily reflect the actual shape, size, or dimensions of the system or illustrated components.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

While the following is primarily directed to a companion or coupler device for use with a reusable optical image endoscope, it should be understood that the features of the presently described coupler device may be readily adapted for use with a variety of reusable or disposable endoscopes, endoscopic instruments and other devices.

The term "endoscope" as used herein refers generally to any scope used on or in a medical application, which includes a body (human or otherwise) and includes, for example, a laparoscope, duodenoscope, endoscopic ultrasound scope, arthroscope, colonoscope, bronchoscopes, enteroscope, cystoscope, laparoscope, laryngoscope, sigmoidoscope, thoracoscope, cardioscope, and saphenous vein harvester with a scope, whether robotic or non-robotic.

When engaged in remote visualization inside the patient's body, a variety of scopes are used. The scope used depends on the degree to which the physician needs to navigate into the body, the type of surgical instruments used in the procedure and the level of invasiveness that is appropriate for the type of procedure. For example, visualization inside the gastrointestinal tract may involve the use of endoscopy in the form of flexible gastroscopes and colonoscopes, endoscopic ultrasound scopes (EUS) and specialty duodenum scopes with lengths that can run many feet and diameters that can exceed 1 centimeter. These scopes can be turned and articulated or steered by the physician as the scope is navigated through the patient. Many of these scopes include one or more working channels for passing and supporting instruments, fluid channels and washing channels for irrigating the tissue and washing the scope, insufflation channels for insufflating to improve navigation and visualization and one or more light guides for illuminating the field of view of the scope.

Smaller and less flexible or rigid scopes, or scopes with a combination of flexibility and rigidity, are also used in medical applications. For example, a smaller, narrower and much shorter scope is used when inspecting a joint and performing arthroscopic surgery, such as surgery on the shoulder or knee. When a surgeon is repairing a meniscal tear in the knee using arthroscopic surgery, a shorter, more rigid scope is usually inserted through a small incision on one side of the knee to visualize the injury, while instruments are passed through incisions on the opposite side of the knee. The instruments can irrigate the scope inside the knee to maintain visualization and to manipulate the tissue to complete the repair

Other scopes may be used for diagnosis and treatment using less invasive endoscopic procedures, including, by way of example, but not limitation, the use of scopes to inspect and treat conditions in the lung (bronchoscopes), mouth (enteroscope), urethra (cystoscope), abdomen and peritoneal cavity (laparoscope), nose and sinus (laryngoscope), anus (sigmoidoscope), chest and thoracic cavity (thoracoscope), and the heart (cardioscope). In addition, robotic medical devices rely on scopes for remote visualization of the areas the robotic device is assessing and treating.

These and other scopes may be inserted through natural orifices (such as the mouth, sinus, ear, urethra, anus and vagina) and through incisions and port-based openings in the patient's skin, cavity, skull, joint, or other medically indicated points of entry. Examples of the diagnostic use of endoscopy with visualization using these medical scopes includes investigating the symptoms of disease, such as maladies of the digestive system (for example, nausea, vomiting, abdominal pain, gastrointestinal bleeding), or confirming a diagnosis, (for example by performing a biopsy for anemia, bleeding, inflammation, and cancer) or surgical treatment of the disease (such as removal of a ruptured appendix or cautery of an endogastric bleed).

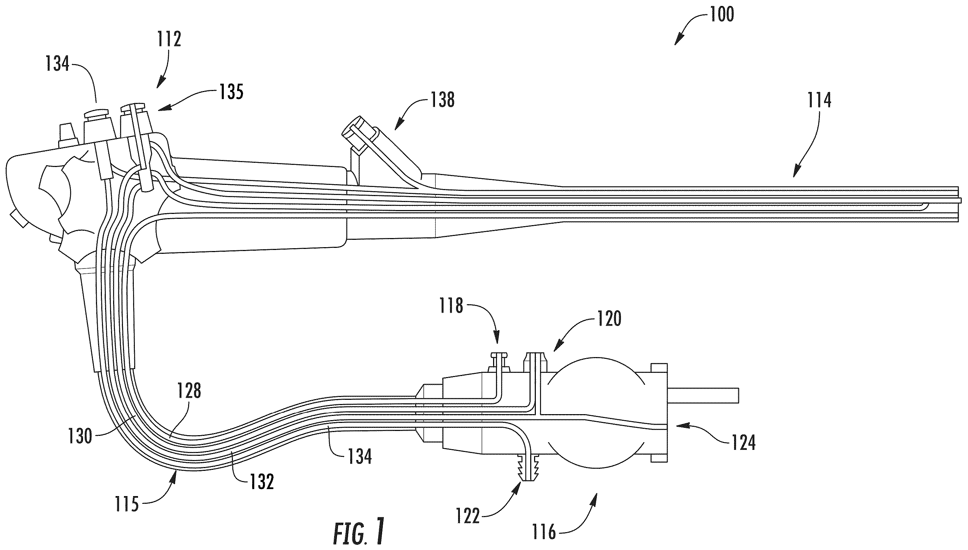

Referring now to Fig. 1, a representative endoscope 100 for use with devices described herein includes a proximal handle 112 adapted for manipulation by the surgeon or clinician coupled to an elongate shaft 114 adapted for insertion through a natural orifice or an endoscopic or percutaneous penetration into a body cavity of a patient. Endoscope 100 further includes a fluid delivery system 116 coupled to handle 112 via a universal cord 115. Fluid delivery system 116 may include a number of different tubes coupled to internal lumens within shaft 114 for delivery of fluid(s), such as water and air, suction, and other features that may be desired by the clinician to displace fluid, blood, debris and particulate matter from the field of view. This provides a better view of the underlying tissue or matter for assessment and therapy. In the representative embodiment, fluid delivery system 116 includes a water-jet connector 118, water bottle connector 120, a suction connector 122 and an air pipe 124. Water-jet connector 118 is coupled to an internal water-jet lumen 126 that extends through handle 112 and elongate shaft 114 to the distal end of endoscope 100. Similarly, water jet connector 118, water bottle connector 120, suction connector 122 and air pipe 124 are each connected to internal lumens 128, 130, 132, 134 respectively, that pass through shaft 114 to the distal end of endoscope 100.

Endoscope 100 may further include a working channel (not shown) for passing instruments therethrough. The working channel permits passage of instruments down the shaft 114 of endoscope 100 for assessment and treatment of tissue and other matter. Such instruments may include cannula, catheters, stents and stent delivery systems, papillotomes, wires, other imaging devices including mini-scopes, baskets, snares and other devices for use with a scope in a lumen.

Proximal handle 112 may include a variety of controls for the surgeon or clinician to operate fluid delivery system 116. In the representative embodiment, handle 112 include a suction valve 135, and air/water valve 136 and a biopsy valve 138 for extracting tissue samples from the patient. Handle 112 will also include an eyepiece (not shown) coupled to an image capture device (not shown), such as a lens and a light transmitting system. The term "image capture device" as used herein also need not refer to devices that only have lenses or other light directing structure. Instead, for example, the image capture device could be any device that can capture and relay an image, including (i) relay lenses between the objective lens at the distal end of the scope and an eyepiece, (ii) fiber optics, (iii) charge coupled devices (CCD), (iv) complementary metal oxide semiconductor (CMOS) sensors. An image capture device may also be merely a chip for sensing light and generating electrical signals for communication corresponding to the sensed light or other technology for transmitting an image. The image capture device may have a viewing end - where the light is captured. Generally, the image capture device can be any device that can view objects, capture images and/or capture video.

In some embodiments, endoscope 100 includes some form of positioning assembly (e.g., hand controls) attached to a proximal end of the shaft to allow the operator to steer the scope. In other embodiments, the scope is part of a robotic element that provides for steerability and positioning of the scope relative to the desired point to investigate and focus the scope.

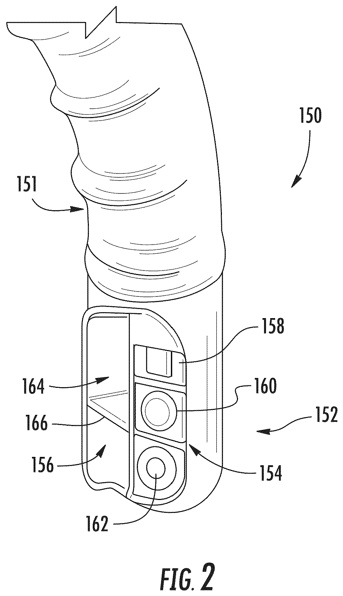

Referring now to Fig. 2, a distal end portion of a side viewing endoscope 150 (e.g., a duodenoscope or EUS) will now be described. As shown, scope 150 includes an elongate flexible shaft 151 with a distal end portion 152 having a viewing region 154 and an instrument region 156, both of which face laterally or to the side of the longitudinal axis of shaft 151. Viewing region 154 includes an air nozzle port 158, a camera lens 160 and a light source 162 for providing a view of the surgical site in the patient. Instrument region 156 includes an opening 164 coupled to a working channel (not shown) within shaft 151 of scope 150. Opening 164 is configured to allow passage of instruments from the working channel of scope 150 to the surgical site. Scope 150 also preferably includes an articulation mechanism for adjusting the angle that the instruments pass through opening 164. In the exemplary embodiment, the articulation mechanism comprises an elevator 166, although it will be recognized by those skilled in the art that the articulation mechanism may include a variety of other components designed to articulate the instrument angle, such as a cable extending through shaft 151 or the like.

Camera lens 160 may comprise one or more optical lenses disposed along an optical path to provide an image of an object at the distal end of endoscope 150. At least one of the optical lens is a fluid or liquid lens that comprises one or more fluids enclosed within a chamber having substantially clear proximal and distal surfaces to allow light to pass through the fluid(s) within the chamber. The focal length of the fluid lens may be adjusted by changing the shape or the index distribution of the fluid. In certain embodiments, the fluid lens is configured such that the surface profiles or radius of curvature of the fluid(s) determines the focal length of the optical lens. By altering the surface profile or radius of curvature of the fluid(s), the focal length of the lens can be adjusted. The surface profile of the fluid(s) may be adjusted by the application of energy directly to, or through, the fluid(s), or indirectly to another mechanism that adjusts the pressure, volume or other characteristics of the fluid.

The lens system may include one or more fluid filled or adjustable lenses and one or more rigid or fixed lenses. The rigid lenses each contribute a constant optical power while the fluid filled lenses may adjust their optical power to adjust the overall optical power of the lens system. Like traditional optical lenses made from glass, liquid lenses are single optical elements, but are composed of an optical liquid material that can change its shape. The focal length of a glass lens is dependent on the material it is made from and its radius of curvature. The same basic principle applies to liquid lenses, though liquid lenses are unique in that they can change their focal length by altering their radius of curvature or the index distribution of the liquid. This change in radius may be electronically controlled and rapidly changed on the order of milliseconds.

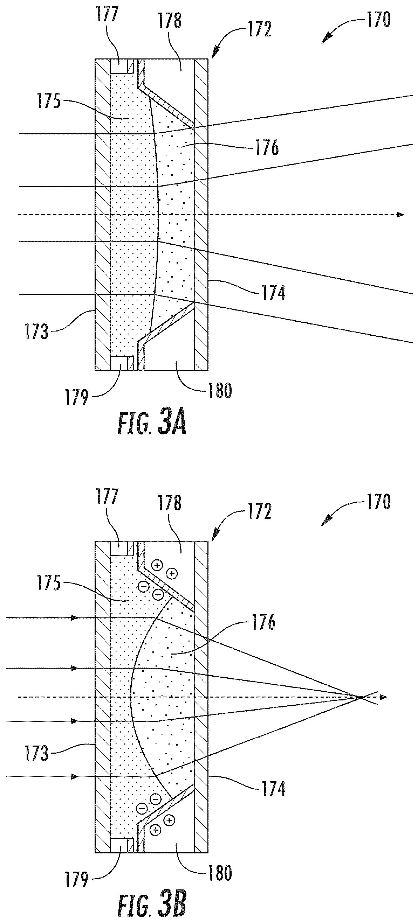

Referring now to Figs. 3A and 3B, one embodiment of a fluid lens 170 will be described. As shown in Fig. 3A, fluid lens 170 includes a housing 172 having proximal and distal surfaces 173, 174 comprising a substantially clear material, such as glass, clear plastic or other suitable material that allows light to pass therethrough. First and second fluids 175, 176 are housed together within housing 172. First fluid 175 preferably comprises a material that will change shape upon the application of energy, such as an electric current, electromagnetic field, electrostatic charge or the like. Second fluid 176 preferably comprises a material that will not mix with first fluid 175 (i.e., first and second fluids 175, 176 are immiscible), but will fill any void or empty space within housing 172. A number of suitable fluids can be used for first and second fluids 175, 176. In a preferred embodiment, first fluid 175 comprises water and second fluid 176 comprises a non-conductive oil.

Lens 170 further comprising first and second electrodes 177, 178 that are coupled to leads, connectors or other coupling elements that are positioned to couple with the separate coupling elements of a removable coupler device 10 (described in more detail below). Alternatively, electrodes 177, 178 may be coupled to one or more connectors that extend through the shaft of endoscope 100. In this embodiment, the focal length of lens 170 may be controlled directly on the handle of endoscope 170, as discussed in more detail below. Electrodes 177, 178 are configured to apply a voltage across the interface between first and second fluids 175, 176. Lens 170 may further include third and fourth electrodes 179, 180 on the opposite side of housing 172.

As shown in Fig. 3B, applying a voltage at the interface between fluids 175, 176 changes the shape and/or size of first fluid 175, which causes second fluid 176 to fill the void left by the displacement of first fluid 175. The change in the shape of fluid 176 alters the curvature and the focal length of lens 170. Applying more voltage increases the overall curvature of second fluid 176 and increases the optical power of lens 170. Applying less voltage decreases the curvature of second fluid 176 and decreases the optical power of lens 170.

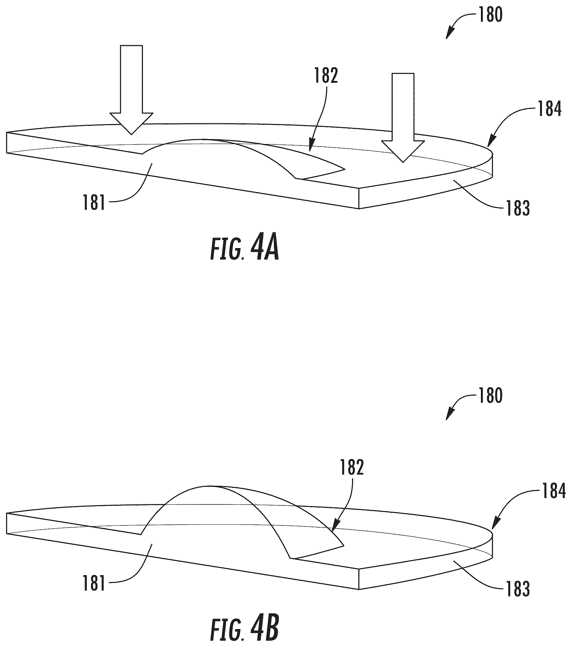

Referring now Figs. 4A and 4B, another embodiment of a fluid-filled lens 180 comprises a container 182 filled with a fluid, such as water, oil, air, or any suitable fluid that allows light to pass therethrough. Container 182 includes a curved chamber 181 and a fluid reservoir 183. Lens 180 further includes a membrane 184 that encloses and seals container 182. An actuator (not shown) is coupled to membrane 184 and configured to exert a pressure on membrane 184. Alternatively, the actuator may exert pressure directly on fluid reservoir 183 or curved chamber 181. The actuator may be driven by a variety of energy sources known to the art, such as electric current, magnetic energy, mechanical, acoustic energy and the like. As membrane 184 exerts more pressure on container 182, the fluid is driven from fluid reservoir 183 to chamber 181, thereby increasing the curvature of lens 180 (see Fig. 4B). Removing this pressure causes the fluid to displace from chamber 181 into reservoir 183, thereby decreasing the curvature of lens 180 (Fig. 4A).

Of course, it will be recognized by those skilled in the art that devices described herein may incorporate a variety of other fluid-filled or liquid lenses. For example, the shape of the fluid may be changed through other mechanisms known in the art, such as acousto-optic tuning, mechanical tuning, piezoelectric and the like. Alternatively, the fluid lens may include two or more fluids with different indexes of refraction. Varying the volume or shape of one of the fluids relative to the other(s) changes the focal length of the length.

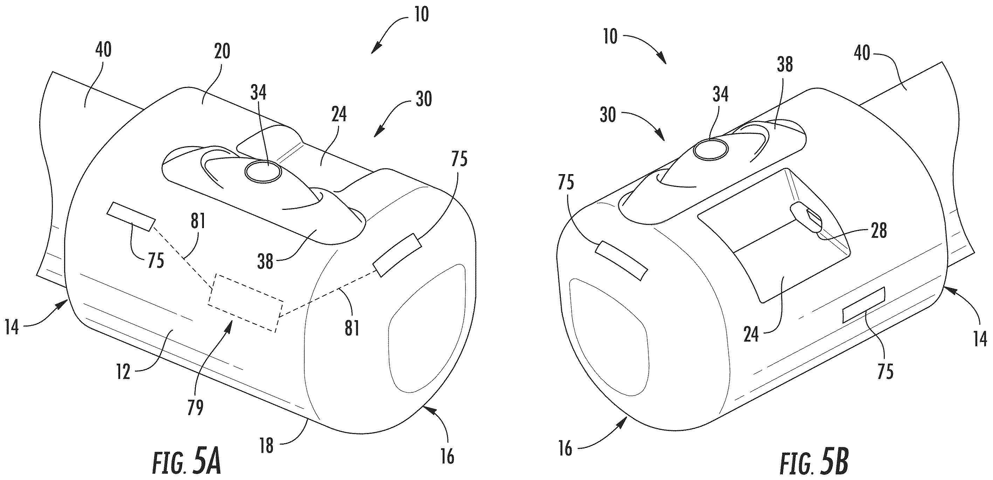

Figs. 5A and 5B illustrate an exemplary embodiment of a coupler device 10. The coupler device 10 serves as an accessory component for currently existing endoscopes. The device seals and covers infection prone areas of the scope to prevent ingress of debris, fluid, or other unwanted matter that could lead to bacterial contamination and decreased performance of the scope.

In certain embodiments, the coupler device 10 provides a flexible working channel for instruments to be inserted into the scope. The flexible working channel can be angularly adjustable with ease. As shown, in the preferred embodiments, the coupler device 10 may be used with a duodenum scope 40 or other side-viewing scope instrument. It is understood, of course, that the coupler device 10 may be adapted for use with end viewing scopes as well. In addition, the coupler device 10 can be used with all types of scopes for different medical applications. The duodenum scope 40 shown here is merely for illustrative purposes.

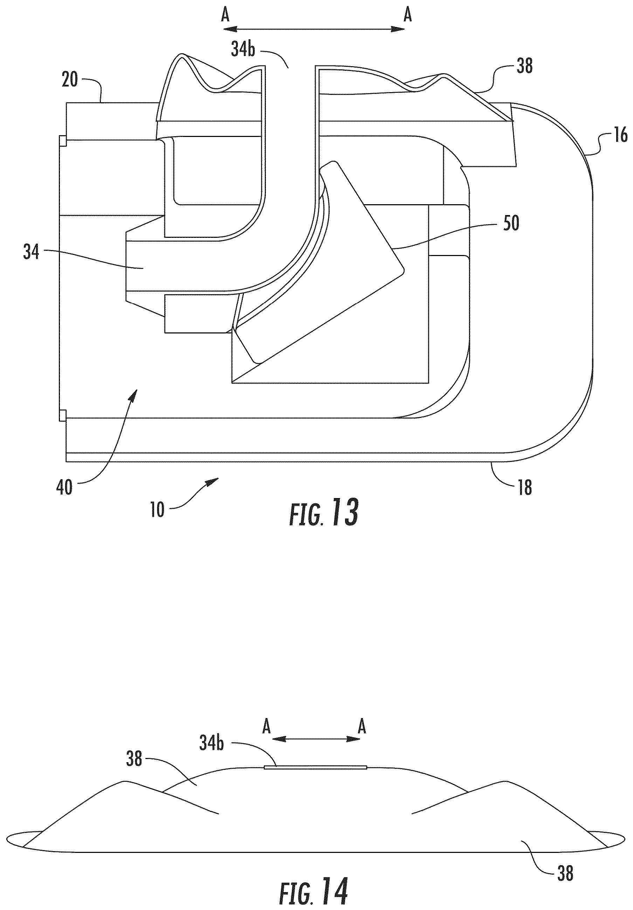

As FIGS. 5A and 5B illustrate, the coupler device 10 may comprise a main body 12, proximal end 14 and distal end 16, lower surface 18 and upper surface 20. The proximal end 14 attaches onto a working end of a duodenum scope 40, extending the working end portion of the scope 40. The upper surface 20 may include a lens and light guide 24 and a scope washer opening 28, which is used to push fluid across the scope camera to wash debris off the camera and is also used to push air across the camera to dry the camera and insufflate the patient's gastrointestinal tract. Upper surface 20 may further include an open area over lens and light guide 24 and scope washer opening 28 to facilitate viewing the surgical site and to allow egress of fluid from scope washer opening 28 into the surgical site (and/or egress of air that may be passed over light guide 24 to dry the camera or that may be passed into the surgical site to insufflate a portion of the site). In addition, the upper surface 20 includes a flexible working channel region 30 that includes a flexible working channel extension 34 that is surrounded by a flexible membrane 38 (see FIG. 14). This flexible membrane 138 serves as a protective hood or covering for the working end of the coupler device 10, providing for flexible articulation while sealing out debris, fluid, bacteria or other unwanted matter.

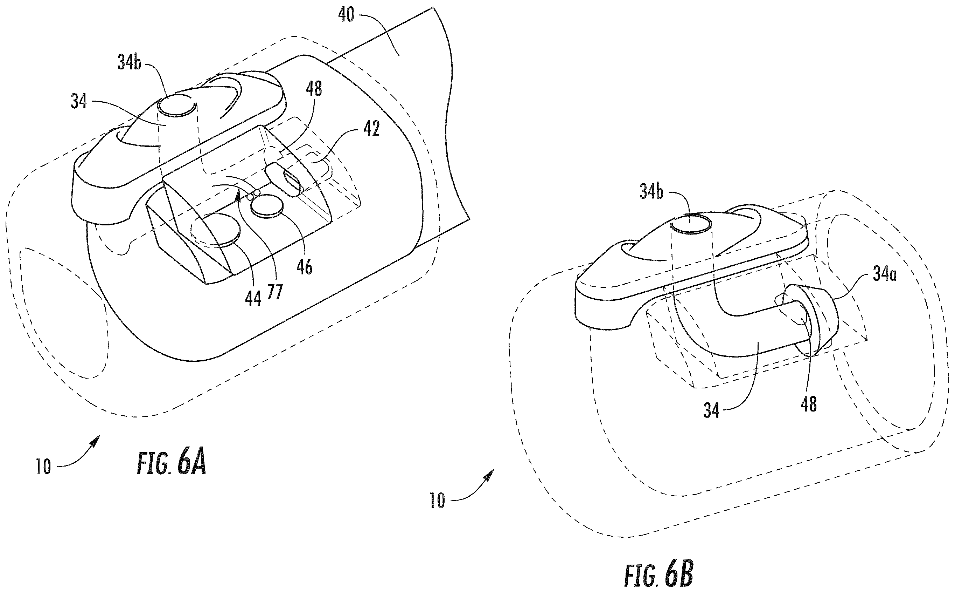

As shown in FIGS. 6A and 6B, the duodenum scope 40 may comprise a light guide 44, an optical lens 46 and a washer opening 48. Optical lens 46 may include more than one lens extending along the optical path and preferably at least one of these lenses is an adjustable fluid-filled lens, such as those described above. The coupler device 10 cooperates with each of these components of the scope 40 to provide a fully functioning scope. The coupler device 10 does not interfere with the scope's ability to emit a clear image, but instead reduces the risk of contamination with each use. This benefit is achieved by providing a coupler device 10 which attaches to the working end components of the scope 40, and seals around the working end.

Referring again to FIG. 5A, coupler device 10 further includes an actuator 79 within main body 12 configured to adjust the focus of optical lens 46. The actuator may include, for example, a controller, a power supply, such as a battery or other suitable source of power, and one or more coupling elements for operatively coupling either directly to optical lens 46 or indirectly through another element within the endoscope. The power supply provides energy to the optical lens 46 through the coupling elements in order to adjust the surface profile or curvature of the lens, thereby adjusting the focus of the lens (as discussed above). The controller operates to control the power supply and thereby control the energy applied to optical lens, as discussed in more detail below.

The coupler device allows the operator to adjust the optical lens to obtain additional positive power correction as needed to view objects at further distances (i.e., focus up close or zoom in on objects without moving the endoscope). Alternatively, the power may be corrected automatically as part of a feedback control loop. In addition the coupler device allows the optical lens to be adjusted continuously over a desired power range. For example, the focal length of the lens may be adjusted to substantially match the distance between a distal end of the endoscope and a desired object, allowing the operator to move the endoscope closer or further away from the object while maintaining focus.

In certain embodiments, the controller, power supply, the coupling elements or another connector within the actuator may be configured to automatically adjust the curvature of lens to refocus the lens on an object as the endoscope is moved within the patient. In other embodiments, the actuator may include a controller that allows the operator to manually adjust the actuator and the focal length of the optical lens when desired. The controller may be controlled from a user input on the handle of the endoscope (i.e., through a connector that extends through the endoscope to the coupler device). Alternatively, the actuator may be controlled wirelessly through a signal that is delivered externally from the patient to the coupler device. For example, if the operator wishes to zoom in on an object without moving the endoscope, the user input can be used to control the actuator and adjust the focus of the optical lens.

In other embodiments, the power supply may be located externally to the coupler device and/or the patient and the power or energy for adjusting the optical lens is delivered wirelessly to the actuator in the coupler device. In one such embodiment, the actuator comprises a receiver configured to wirelessly receive energy from a source of energy and to transmit the energy to the optical lens. Alternatively or in addition, the receiver may be configured to receive electrical signals (e.g., command inputs) from a remote source to allow the operator to control the actuator. The receiver may be disposed within the main body or on the outer surface of the coupler device. The source of energy and/or the controller may be located remotely from the coupler device, such as exterior to the patient. The energy may comprise an electromagnetic field, an electromagnetic radiation or any other suitable energy that may be transmitted wirelessly from the source of energy to the actuator.

The actuator within coupler device may be configured to articulate an instrument passing through a working channel in the endoscope. In this embodiment, the actuator includes an articulating element positioned to either directly contact the instrument as it passes through coupler device 10, or positioned to articulate a flexing working channel extension with coupler device 10 (discussed in more detail below). The actuator may be configured to both articulate the instrument and to supply energy to the optical lens. In certain embodiments, the coupler device comprises two such actuators that may be powered by a source of energy within coupler device 10, or externally through wireless energy transmission.

As shown in Figs. 5A and 5B, coupler device 10 may include one or more receivers 75 on, or within, an outer surface of main body 12 and coupled to actuator 79 via one or more connectors 81. Receivers 75 are preferably configured to receive energy and/or signals from a remote source, as discussed in more detail below. In one embodiment, coupler device 10 includes at least two receivers located on a distal portion of the outer surface and one or more of the side or lateral portions of the outer surface. In other embodiments, receivers 75 may also be located on upper and lower portions of the outer surface. Alternatively, one or more receivers 75 may be located within main body 12.

As shown in FIG. 6A, actuator 79 is coupled to one or more coupling elements 77. Coupling elements 77 are positioned such that, when proximal end 14 of coupler device 10 is attached to the working end of an endoscope, coupling elements 77 are in operational proximity to optical lens 46. Operational proximity as defined herein means that the coupling elements 77 are positioned so as to apply energy to optical lens to alter the focal length of the lens. Coupling elements 77 may be in direct contact with the lens 46, adjacent to the lens, or in direct contact or adjacent to another coupling element or actuator in the endoscope. In the latter configuration, coupling elements 77 transmit energy to the other coupling element or actuator, which then functions to transmit the energy to lens 46 and/or to an actuator that alters the curvature of lens 46.

In one embodiment, coupling elements 77 comprise one or more electrodes positioned to applying a voltage, electromagnetic field, electrostatic charge or the like to optical lens 46 when coupler device 10 is attached to the endoscope. As described above, this electrical signal changes the shape of one or more fluid(s) within lens 46, thereby altering its curvature and focal length. In another embodiment, coupling elements 77 comprise one or more connectors or leads that are configured to transmit energy to the optical lens 46. The connectors may be configured to transmit an electrical signal to other connectors within scope 100 or directly to electrodes (not shown) within scope 100.

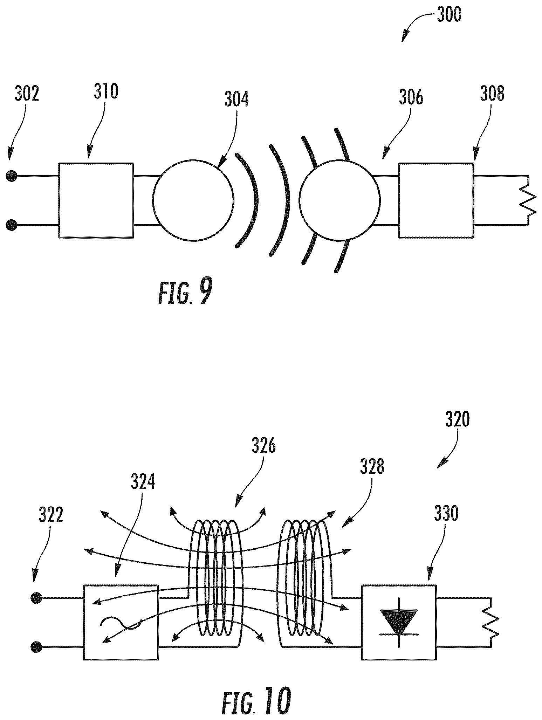

Referring now to Figs. 9, 10, 11A and 11B, several embodiments for wirelessly transferring energy and/or electrical signals to the actuator within coupler device 10 will now be described. As shown in Fig. 9, an energy delivery system 300 comprises a power source 302, a transmitter 304, such as an antenna, magnetic or electric field generator or other suitable coupling device, a receiver 306 for receiving the energy, and a converter 308 for converting the energy supplied to receiver 308 into another suitable form for use in varying the curvature or shape of the optical lens on the endoscope. Coupler device 10 may further include an energy storage unit (such as a capacitor) for storing transmitted energy that may be used by the operator when desired. Receiver 306 and converter 308 may be located together within main body 12 of coupler device 10. Alternatively, one or more receivers 306 may be located on outer surface 17 of coupler device 10 and coupled to converter 308 with connectors (not shown). Energy delivery system 300 may also include an energy generator or convertor 310 for converting the energy generated by power source 302 into a suitable form for transmitting by transmitter 304.

In one embodiment, energy delivery system 300 comprises a far-field or radiative system wherein energy or power is transferred by beams of electromagnetic radiation, such as microwaves, radio frequency, laser beams or the like. Power source 302 and/or converter 310 converts energy or power into a time-varying or oscillating electromagnetic field. Antenna 304 radiates the electromagnetic radiation to receiver 306. There may be several antennae at various orientations in an external control unit (not shown) to enhance coupling efficiency in various orientations of coupler device 10. Receiver 306 and/or converter 308 then converts the energy back to a suitable form, such as a DC or AC electric current, for delivery to the optical lens.

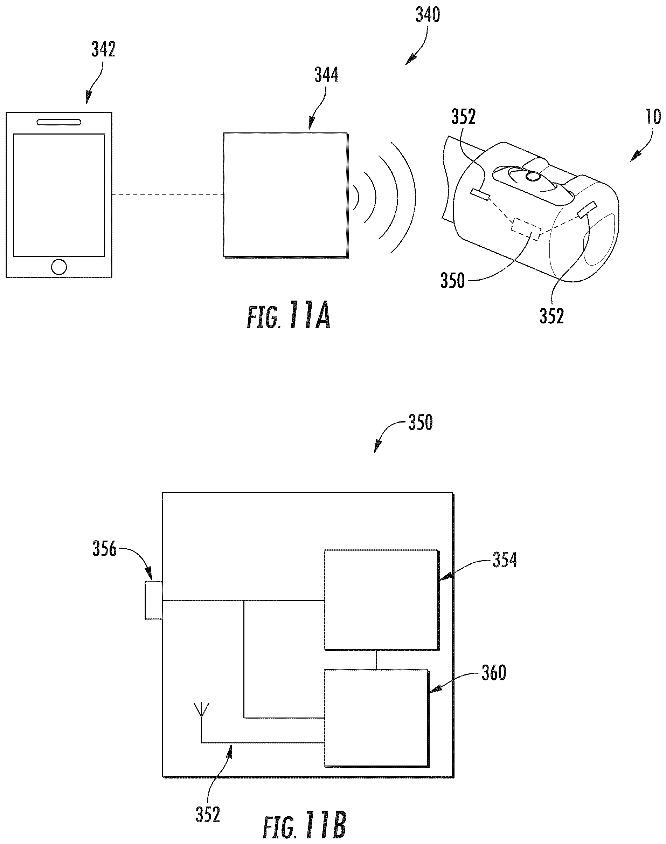

Antenna 304 may also deliver electrical signals to the actuator within coupler device 10 to control the application of energy to the optical lens (discussed in more detail below). Energy delivery system 300 may further include a controller (see Fig. 11A) with user inputs that allows the operator to control the transmission of power , energy and/or electrical signals to coupler device 10.

Fig. 10 illustrates another embodiment of an energy delivery system 320 that primarily uses a near field or non-radiative mechanism for transmitting power or energy to coupler device 10. Energy delivery system 320 comprises a power source 322, an oscillator 324 for converting the energy into a time-varying or oscillating electromagnetic field and one or more magnetic coils 326. Magnetic coils 326 transmit the electromagnetic field to one or more magnetic coils 328 on coupler device 10 through inductive coupling. Magnetic coils 326, 328 together form a transformer that induces an alternative EMF voltage which creates an alternating current. The induced alternating current may drive the load directly, or be rectified by a rectifier 330 on coupler device to DC current that can be supplied to the optical lens.

In an alternative embodiment, magnetic coils 326, 328 may be replaced with electrodes (not shown). In this embodiment the electrodes transmit an electric field using capacitive coupling techniques that are well known in the art.

It should be understood that the methods and devices described herein are not limited to the above embodiments for wirelessly transmitting energy and/or electrical signals to coupler device 10. For example, wireless electrical signals can be transferred to coupler device 10 with radio waves (e.g., Bluetooth), acoustic energy, infrared or ultrasonic remote control, free-space optical communication, electromagnetic induction and the like.

Referring now to Figs. 11A and 11B, a control system 340 for controlling an actuator 350 within coupler device 10 will now be described. As discussed above, the actuator in coupler device 10 may be configured to automatically refocus the optical lens as necessary during the procedure, in which case control system 340 may not be necessary. In these embodiments, actuator 350 may include an autofocus light sensor (not shown) and a controller that evaluates signals from the autofocus light sensor. The controller then automatically supplies energy to the lens, as necessary, to adjust the focal length of the lens until the image is in focus. In certain embodiments, however, control system 340 allows the operator to control the operation of actuator 350 to change the focal length of the optical lens as desired, either by controlling the delivery of energy to actuator 350, or controlling the delivery of energy from actuator 350 to the optical lens.

As shown in Fig. 11A, control system 340 may include a user interface device 342 and an external power source and transmitter 344 for transmitting signals, energy and/or power to coupler device 10. User interface device 342 includes one or more user controls, such as knobs, switches, dials, touch-screen or the like, for allowing an operator to control transmitter 344 and transmit signals and/or energy to coupler device 10. In some embodiments, user interface device 342 is located on handle 112 of endoscope 100 (see Fig. 1) and may simply include a button or slide switch to provide a user-friendly interface for controlling transmitter 344. In these embodiments, interface device 342 may be coupled to connectors that extend through the shaft of the endoscope to the optical lens and/or the coupler device.

Referring now to Fig. 11B, actuator 350 resides within, or on, coupler device 10. Actuator 350 may include a receiver 352, such as an antenna, magnetic coil, electrode, or the like, and a control until 354 for controlling the application of energy to one or more coupling elements 356, such as electrodes, leads or other connectors. As discussing above, coupling elements 356 are positioned to be in operational proximity to an optical lens of an endoscope when coupler device 10 is attached thereto. Control unit 354 may also be wirelessly coupled to user interface device 342 or transmitter 344 such that the user can control the application of energy to coupling elements 356.

In certain embodiments, actuator 350 further includes an internal power source 360, such as a battery or the like, for generating energy within actuator 350. In these embodiments, energy source and transmitter 344 may not be necessary to operate actuator 350. Power source 360 generates energy and transmits the energy to coupling elements 356. This generation and transmission of energy may be automatic (as discussed above), or it may be controlled by the operator through user interface device 344.

In other embodiments, actuator 350 only includes a receiver 352, a converter (not shown) and coupling elements 356. In these embodiments, energy is transmitted to receiver 352, which automatically applies the energy through the converter and coupling elements 356 to the optical lens. Thus, a separate controller is not required in actuator 350 in these embodiments.

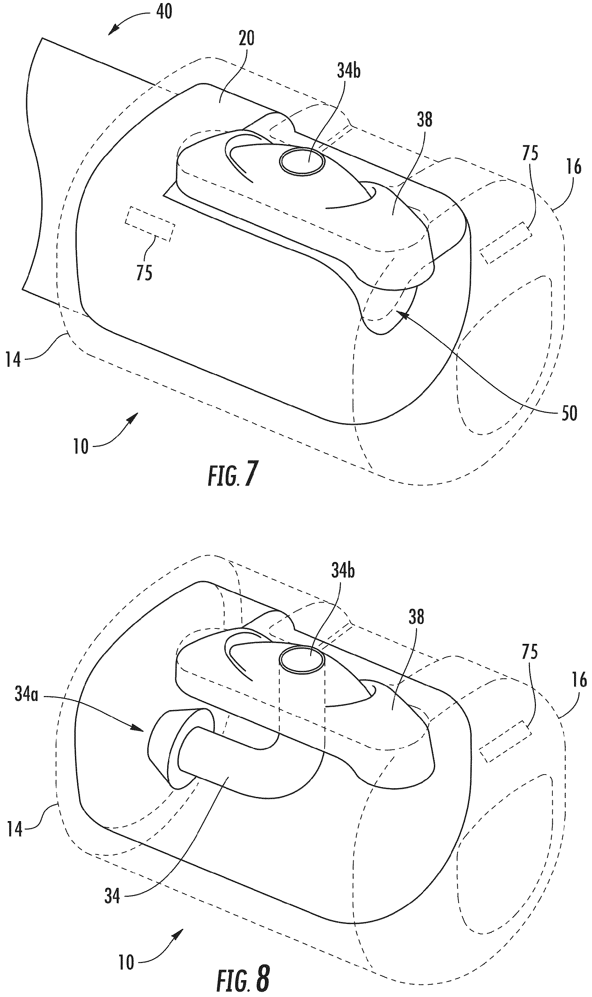

As further shown in FIGS. 5A, 5B, 6A, 6B, 7 and 8, the coupler device 10 provides an extension of the scope's working channel 42. The working channel extension 34 of the coupler device 10 in FIGS. 5A and 5B is flexible and may contact the scope's working channel 42 by a sealed connection, as shown in FIG. 8, at the proximal end 34a of the working channel extension. The distal end 34b of the working channel extension 34 serves as an exit portal for instruments to pass through the scope 40 to reach different areas of the body.

Additionally, the coupler device 10 provides a further seal around the elevator 50 of the scope. Because the coupler device 10 seals the elevator 40, risk of debris influx, fluids, bacteria and other matter build up behind the elevator and working channel is reduced significantly. This influx of debris, bacteria and other matter is believed to be the reason for drug resistant infections with current scopes today. While preventing influx, the coupler device 10 advantageously maintains flexibility to move the working channel extension 34.

In use, the scope's working channel extension 34 permits passage of instruments down the scope working channel 42 and through and out the working channel extension 34 of the device 40 for assessment and treatment of tissue and other matter. Such instruments may include cannula, catheters, stents and stent delivery systems, papillotomes, wires, other imaging devices including mini-scopes, baskets, snares and other devices for use with a scope in a lumen. This working channel extension 34 is flexible enough that the elevator 50 of the scope 40 can raise and lower the working channel extension 34 so that instruments can be advanced down and out of the working channel extension distal end (or exit portal) 34b of the scope 40 at various angles, or be raised and lowered by a cable or other means to articulate the working channel extension 34.

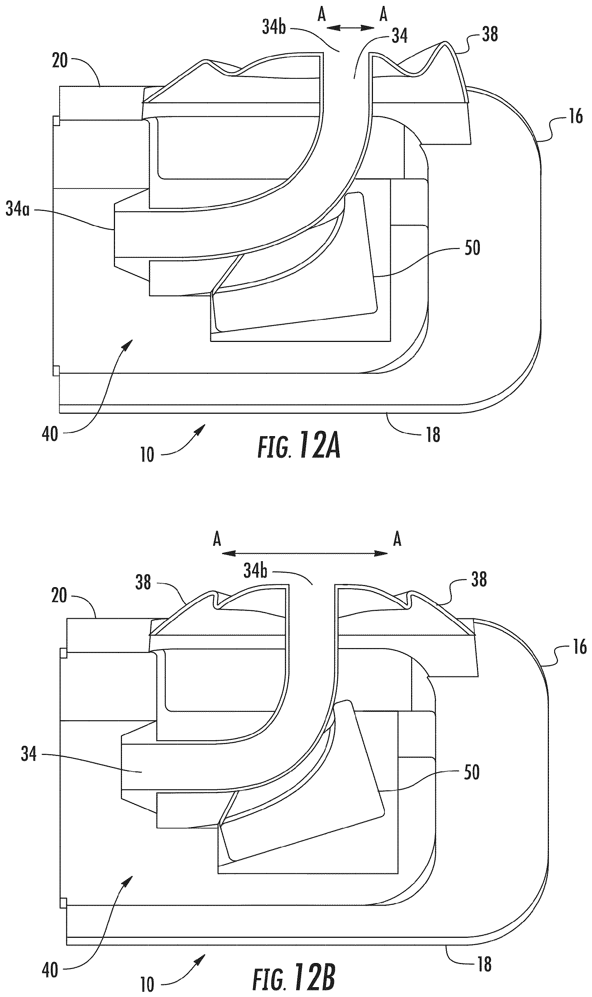

As FIGS. 12 to 14 illustrate, in use when the elevator 50 of the scope 40 is actuated, the flexible working channel extension 34 of the coupler device moves or adjusts to this actuation, along the direction A-A. In FIG. 12A, the elevator 50 is raised slightly, creating a hinged ramp or shoulder that pushes the working channel extension 34 a corresponding angle and shifts the exit portal or distal end 34b of the working channel extension to the left. In FIG. 12B the elevator is raised higher than in FIG. 12A, such that the distal end 34b of working channel extension 34 is likewise shifted further to the left in comparison to FIG. 12A, while FIG. 13 shows the elevator 50 raised even higher and the distal end 34b of working channel extension 34 moved to the left even further in comparison to FIGS. 12A and 12B.

Of course, it will be recognized that the instruments passing through the scope may be articulated by a variety of different mechanisms. For example, in some embodiments, the device may have multiple cables so the angle of exit can be articulated in multiple directions, including in different quadrants, unlike with the current endoscope elevators, which can only deflect and therefore redirect an instrument in a single axis due to the limited travel of endoscope elevators, which can only be raised or lowered, but not moved from side to side or articulated into other quadrants. In some embodiments, the cable(s) may be attached directly to the working channel extension or to other devices that can be articulated and cause the working channel extension to change its angle of exit, including, for example, a dowel underneath the working channel extension, but encased in the device that can be advanced forward and backward to move the working channel extension as the cable is advanced and retracted. In some embodiments, the articulation ability of the coupler device may be created with an elevator embedded in the coupler device, which is disposable and therefore thrown away after the procedure.

The articulation ability of the coupler device may also take place with elements that do not involve cables, including for example, piezo electric materials, micro motors, organic semiconductors, and electrically activated polymers. In some embodiments, the articulation ability of the coupler device may also take place with the transfer of force to the working channel extension or an embedded elevator through interlocking connectors that transfer force, wires that twist, slidable sheaths, and memory metals that change shape through the transfer of temperature. These interlocking connectors may be powered by a source of energy within the coupler device, such as a battery or the like, or through wireless energy transmission, as discussed above.

In some embodiments, the device includes a power connector or motors to deliver energy, including electromagnetic energy, to the device to cause a transfer in force to change the angle of exit from the coupler device as an instrument is passed through the device, or in advance of passing an instrument through the device. This transfer of force can include causing the device to rotate as it exits the working channel extension. The device may be navigated and articulated by the user directly, or as part of a robotic system in which the users input is translated through the system through various means, including cables, power connectors, motors, electromagnetic energy, slidable sheaths, haptics, computer-guided and directed input, and other means to direct and guide the device to its intended location, including to specific diagnosis and treatment objectives in a patient, or in non-medical applications, to a desired remote location. A more complete description of different embodiments of a coupler device can be found in commonly-assigned co-pending, U.S. Application Serial No. 15,906,557, filed February 27, 2018, the complete disclosure of which is hereby incorporated herein by reference for all purposes as if copied and pasted herein.

Also, it will be recognized that devices described herein are not limited to the specific coupler device described above. For example, coupler device 10 may be adapted for use with scopes that are actuated by cable and eliminates the need for the elevator component. In this embodiment, the coupler device 10 may include the same structural features as previously described, but further includes a disposable external sheath (not shown) that can receive an interior actuating cable of the scope. A more complete description of this design can be found in commonly-assigned co-pending US application Serial No. 15/746,196, filed July 21, 2016, the complete disclosure of which is incorporated herein by reference for all purposes as if copied and pasted herein.

Coupler device 10 may also include one or more locking elements configured to constrain and/or secure an instrument passed therethrough to ensure that the instrument remains in place at the target site and/or to facilitate instrument exchange. A more complete description of this design can be found in commonly-assigned co-pending US provisional application Serial No. 63/007,461 filed April 9, 2020, the complete disclosure of which is incorporated herein by reference for all purposes as if copied and pasted herein.

Coupler device 10 may further include one or more sensors on the outer surface of the main body of the coupler device. The sensors may be configured to detect a physiological parameter of tissue around the outer surface of the main body of the coupler device. The physiological parameter may include, for example, a temperature of the tissue. a dimension of the tissue, a depth of the tissue, tissue topography, tissue biomarkers, tissue bioimpedance, temperature, PH, histological parameters or another parameter that may be used for diagnosing a medical condition. A more complete description of this design can be found in commonly-assigned co-pending US provisional application Serial No. 63/003,656 filed April 1, 2020, the complete disclosure of which is incorporated herein by reference for all purposes as if copied and pasted herein.

Coupler device 10 may also include one or more optical layers configured to reduce an amount of reflected light from the surface and/or to inhibit condensation of water droplets on the visualization section, or both. Reducing the glare and/or fogging of the visualization section significantly improves the surgeon's view of the target site through the optical coupler. A more complete description of this design can be found in commonly-assigned co-pending US provisional application Serial No. 62/949,238 filed December 17, 2019, the complete disclosure of which is incorporated herein by reference for all purposes as if copied and pasted herein.

In other embodiments, the coupler device 10 may also include a closable port (i.e., self-sealing) that allows for the injection of anti-adhesion, antibacterial, anti-inflammatory or other drug or infusible matter that prevents the adherence or colonization of bacteria on the scope. An applicator may be provided that is integrated into the coupler device 10 with a port for delivery of the infusible matter. Alternatively, the applicator may be separate from the coupler device 10 and applied to the distal end of the scope 40. The infusible matter may include forms of silver, including in a gel or other solution, platinum, copper, other anti-adhesion, antibacterial, anti-inflammatory or other drug or infusible matter that is compatible with the scope and coupler device materials and biocompatible for patient use.

In one exemplary embodiment, the device includes an anti-infective material. In another exemplary embodiment, the device includes an anti-infective coating. In still another embodiment, the device includes a coating that is hydrophobic. In yet another embodiment, the device is superhydrophobic. In even still another embodiment, the device is anti-infective and hydrophobic. Further yet in another embodiment, the device is anti-infective and superhydrophobic. In further still another exemplary embodiment, anti-inflammatory coatings are incorporated into the device. In other embodiments, the anti-inflammatory coating may be hydrophilic.

In one exemplary embodiment, the device 10 may include a silver ion coating. In another embodiment, the device 10 may have a silver hydrogel applied, infused, or made part of the device 10 in the area that covers or goes around the scope elevators. In addition to silver having antimicrobial properties, silver can also conduct electricity. Thus, in still another embodiment, the device 10 may include an electrical wire or other power transmission point to enable the creation of an electric field across the silver ion coating to improve the ability of the silver ion coating to prevent infection. In some embodiments, the electrical wire or other power transmission point may also apply to other antimicrobial and conductive materials, including platinum and copper.

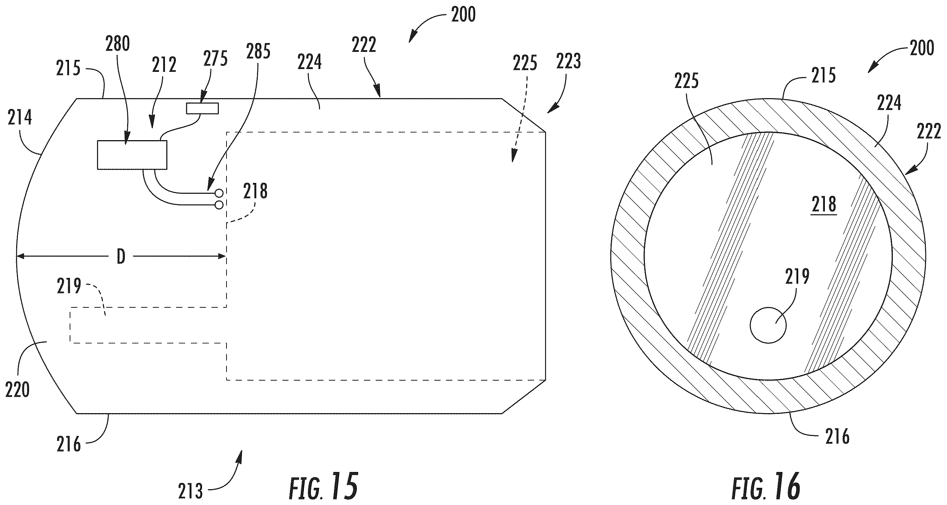

Turning now to Figures 15-17, there is shown an alternative embodiment of an optical coupler 200. The optical coupler 200 includes a visualization section 212 at a distal end 213 of the optical coupler 200. The visualization section 212 has a generally slightly curved, convex outer surface 214 that extends from a first outer side boundary 215 to a second opposite outer side boundary 216 of the optical coupler 200. The outer surface 214 may be constructed to be generally flat, but a curved outer surface 214 is preferable because the curvature helps to clear the field of view by pushing any fluid or matter from the center of the outer surface 214 to the outer boundaries 215, 216. A flat outer surface 214 may be more difficult to clear since the pressure is equal across the entire area of contact and fluid can become trapped between the lens and a surface in which it is desired to view or perform work. A curved outer surface 214 is also preferable to correct any curvature distortion created by an objective lens that may be used in conjunction with the coupler 200.

The optical coupler 200 has a proximal surface 218, and a hollow instrument channel 219 extends from the proximal surface 218 toward the outer surface 214. The hollow instrument channel 219 may be constructed such that the channel 219 does not extend all the way through the visualization section 212 to the outer surface 214. In such a case, a barrier section 220 of material is provided between a distal end 221 of the hollow instrument channel 219 and the outer surface 214 of the optical coupler 200. Alternatively, the instrument channel 219 may extend the full length of the visualization section 212, extending through the optical coupler 200. Such a configuration may allow for the free and unencumbered exchange of instruments. A water tight seal or valve, such as a Tuohy-Borsttype valve, may be employed on the proximal end of the endoscope instrument channel 219 to prevent or minimize air, fluid, and/or foreign matter from flowing through the instrument channel 219.

While an instrument channel 219 is shown in the optical coupler 200 of Figures 15-17, the visualization section 212 may be constructed without an instrument channel 219. In such a case, instruments may be passed directly through the visualization section 212 as the visualization section 212 may be constructed of a material that is self-sealing and elastic enough to permit instruments to be passed through the entire length of the visualization section 212 of the optical coupler 200. An example of an optical coupler 200 without an instrument channel 219 is described in U.S Patent No. 8,905,921 to Titus, the complete disclosure of which is hereby incorporated herein by reference in its entirety for all purposes.

The optical coupler 200 also includes an attachment section 222 connected to and extending away from the visualization section 212. The attachment section 222 is at the proximal end 223 of the optical coupler 200. The proximal end 223 of the optical coupler may be angled to lessen the chance that the optical coupler 200 may catch on any surfaces when the optical coupler 200 is being removed from its environment of use. In the embodiment shown, the attachment section 222 is in the form of a cylindrical wall 224. The proximal surface 218 and the cylindrical wall 224 of the optical coupler 200 define a hollow cylindrical opening 225 of the optical coupler 200 within the sleeve-like cylindrical wall 224.

Optical coupler 200 further includes an actuator 280 configured to adjust the focus of an optical lens within the endoscope. Similar to previous embodiments, the actuator may include, for example, a controller, a power supply, such as a battery or other suitable source of power, and one or more coupling elements for coupling either directly to the optical lens or indirectly through another element within the endoscope. The power supply provides energy to the optical through the coupling elements in order to adjust the surface profile or curvature of the lens, thereby adjusting the focus of the lens (as discussed above)

In certain embodiments, the power supply, the coupling elements or another connector within the actuator may be configured to automatically adjust the curvature of lens to refocus the lens on an object as the endoscope is moved within the patient. In other embodiments, the actuator may include a controller that is wirelessly linked to a user input to allow the operator to manually adjust the actuator when desired. For example, if the operator wishes to zoom in on an object without moving the endoscope, the user input can be used to control the actuator and adjust the focus of the optical lens. The controller may be controlled through a wireless command function or through wireless delivery of energy, as discussed in more detail below. In other embodiments, the power supply may be external to coupler device 10 and the power or energy to operate the actuator is delivered wirelessly to optical coupler 10, as discussed in more detail below.

In certain embodiments, optical coupler 200 further includes one or more receivers 275 on, or within, visualization section 212 and/or attachment section 222. Receivers 275 are preferably configured to receive energy and/or command inputs from a remote source, as discussed above. Receivers 275 are coupled to actuator 280, which is, in turn, coupled to one or more coupling elements 285. Coupling elements 285 are positioned such that, when attachment section 222 of optical coupler 200 is attached to the working end of an endoscope, coupling elements 285 are in operational proximity to optical lens 240 Operational proximity as defined herein means that the coupling elements 285 are positioned so as to apply energy to optical lens to alter the curvature of the lens. Coupling elements 285 may be in direct contact with lens, adjacent to lens, or in direct contact or adjacent to another coupling element or actuator in the endoscope. In the latter configuration, coupling elements 285 transmit energy to the other coupling element or actuator, which then functions to transmit the energy to lens 46 and/or to an actuator that alters the curvature of lens 240.

In one embodiment, coupling elements 285 comprise one or more electrodes positioned to applying a voltage or electrostatic charge to optical lens 240. As described above, this voltage or electric charge changes the shape of one or more fluid(s) within lens 240, thereby altering its curvature. In another embodiment, coupling elements 285 comprise one or more connectors or leads that are configured to transmit energy to optical lens 240.

Optical coupler 200 may further include an ultrasound transducer or sensor (not shown) mounted in either attachment section 222 or visualization section 212. The ultrasound transducer may be a transmitter, receiver or a transceiver. The electrical signal may be transmitted to the ultrasound transducer through a connector that extends through the endoscope, or wirelessly through the patient's body. The transducer measures the time between sending a sound signal and receiving the echo of the sound signal to calculate the distance therebetween. This data can be collected by a processor coupled to the endoscope, or wirelessly directly to the optical coupler 200, to measure depth of tissue and create a 3-D representation of a target area within the patient.

Optical coupler 200, or the endoscope, may also include a laser or other light transmitter (not shown) for transmitting ultraviolet, visible and/or infrared light against target tissue. In this embodiment, sensors 275 are configured to detect the reflected light (i.e., light return times and/or wavelengths) and to transmit signals related to the reflected light to the processor. This data can be used to create a 3-D representation of the target tissue.

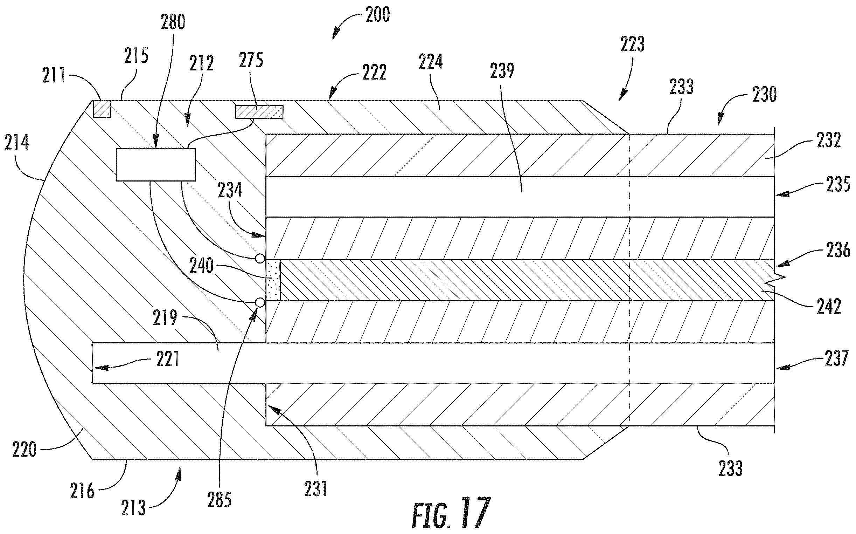

Referring to Figure 17, the optical coupler 200 can be mounted on an endoscope 230. The endoscope 230 has a distal end 231 that is inserted in the hollow cylindrical opening 225 of the optical coupler 200. In one form, the cylindrical wall 224 of the coupler 200 has a diameter one to three millimeters larger than the endoscope 230. The endoscope 230 has a sheath 232 with an outer surface 233 that snugly engages the cylindrical wall 224 of the optical coupler 200. In a non-limiting example, the sheath 232 has an outside diameter of 7-15 millimeters. An end surface 234 of the endoscope 230 sealingly engages the proximal surface 218 of the optical coupler 200.

The endoscope 230 includes a first lumen 235 and a second lumen 236 and a third lumen 237 that extend from the end surface 234 of the endoscope 230 to a proximal end (not shown) of the endoscope. Lumen internal diameters of 2-4 millimeters are typical. A light guide 239 is positioned in the first lumen 235 for transmitting light toward a surface area at or beyond the outer surface 214 of the optical coupler 200.

An object lens 240 is positioned at a distal end of an image carrying fiber 242, and the lens 240 is optically connected to the image carrying fiber 42 for receiving light that has been reflected from the surface area being viewed. The object lens 240 and the image carrying fiber 242 are located in the second lumen 236. As discussed above, lens 240 preferably comprises a fluid lens that can be adjusted through the application of energy from actuator 280.

The third lumen 237 aligns with the hollow instrument channel 219 of the optical coupler 200 when the optical coupler 200 is mounted on the endoscope 230. In the embodiment shown, the instrument channel 219 and the third lumen 237 have the same size inner diameter within a tolerance of ± 5%. The optical coupler 20 can also include a Light Emitting Diode (LED) 211 near the outer surface 214 of the coupler to provide illumination prior to the coupler contacting any fluids, tissue, or structure. The LED 211 may be provided power via a wire (not shown) in the endoscope 230 or from an external source.

The design of the length D for the optical coupler 200 should also take into consideration the characteristics of the materials that compose the coupler 200, such as any possible compression of the coupler 200 when it is held against a surface. For example, if the coupler 200 may be compressed 1 millimeter when held against a surface and the lowest value in the depth of field distance range of the endoscope 230 is 2 millimeters, then the length D should be greater than or equal to 3 millimeters to compensate for this possible compression.

The optical coupler 200 can be formed from a variety of materials. In one version of the optical coupler 200, the optical coupler 200 is molded from a material selected from silicone gels, silicone elastomers, epoxies, polyurethanes, and mixtures thereof. The silicone gels can be lightly cross-linked polysiloxane (e.g., polydimethylsiloxane) fluids, where the cross-link is introduced through a multifunctional silane. The silicone elastomers can be cross-linked fluids whose three-dimensional structure is much more intricate than a gel as there is very little free fluid in the matrix. In another version of the optical coupler 200, the material is selected from hydrogels such as polyvinyl alcohol, poly(hydroxyethyl methacrylate), polyethylene glycol, poly(methacrylic acid) , and mixtures thereof. The material for the optical coupler 200 may also be selected from albumin based gels, mineral oil based gels, polyisoprene, or polybutadiene. Preferably, the material is viscoelastic.

Referring back to Figures 15-17, in the optical coupler 200, the material is optically clear such that the light guide 239 can transmit light through the optical coupler 200 toward a surface area at or beyond the outer surface 214 of the optical coupler 200 and such that the optical coupler 200 is capable of transmitting an optical image of the surface area being viewed back to the lens 240. In one version of the optical coupler 200, the material has a degree of light transmittance greater than 80% based on test standard ASTM D-1003 (Standard Test Method for Haze and Luminous Transmittance of Transparent Plastics). In another version of the optical coupler 200, the material has a degree of light transmittance greater than 90% based on test standard ASTM D-1003. In another version of the optical coupler 200, the material has a degree of light transmittance greater than 95% based on test standard ASTM D-1003. In another version of the optical coupler 200, the material has a degree of light transmittance greater than 98% based on test standard ASTM D-1003. Preferably, the material has an optical absorption of less than 0.1% in the visible light range, and more preferably the material has an optical absorption of less than 0.01% in the visible light range. The material has an index of refraction of about 1.3 to about 1.7, and preferably, the index of refraction of the material matches the index of refraction of the light guide 39, or is as low as possible.

The optical coupler 200 may also be coated with different materials to reduce the amount of adherence properties. Additionally, some coatings of the optical coupler 200 improve with light reflections, as discussed above. Sample coatings that may be used on the optical coupler include thermoplastic film polymer based on p-xylylene such as Parylene C, which is an optically clear biocompatible polymer having abrasion resistant and hydrophobic properties.

Optical coupler 200 may have a variety of other configurations and features. A more complete description of these configurations and features can be found in U.S Patent No. 8,905,921 to Titus, the complete disclosure of which has already hereby incorporated herein by reference as if copied and pasted herein.

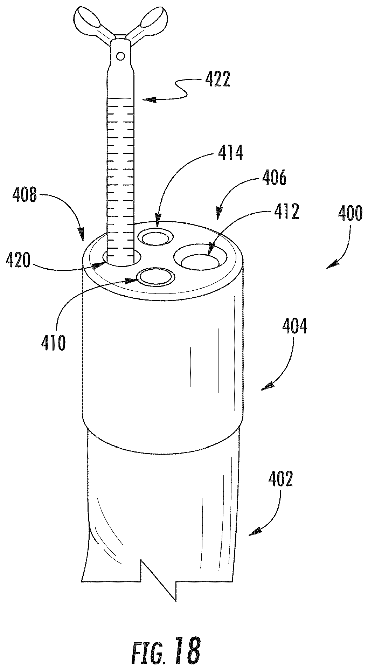

Referring now to Fig. 18, a distal end portion of a forward or end-viewing endoscope 400 (e.g., a colonoscope) will now be described. As shown, scope 400 includes an elongate flexible shaft 402 with a distal end portion 404 having a viewing region 406 and an instrument region 408, both of which face forward or towards the distal end of the longitudinal axis of shaft 402. Viewing region 406 includes a fluid port 410, a camera lens 412 and a light source 414 for providing a view of the surgical site in the patient. As with previous embodiments, lens 412 may comprise include one or more fluid filled adjustable lenses and one or more rigid or fixed lenses. Instrument region 408 includes an opening 420 coupled to a working channel (not shown) within shaft 402 of scope 400. Opening 420 is configured to allow passage of instruments 422 from the working channel of scope 400 to the surgical site. Scope 400 also preferably includes an articulation mechanism for adjusting the angle that the instruments pass through opening 420. In the exemplary embodiment, the articulation mechanism comprises a cable (not shown) extending through shaft 402, although it will be recognized by those skilled in the art that the articulation mechanisms may include a variety of other components designed to articulate the instrument angle, such as an elevator or the like.

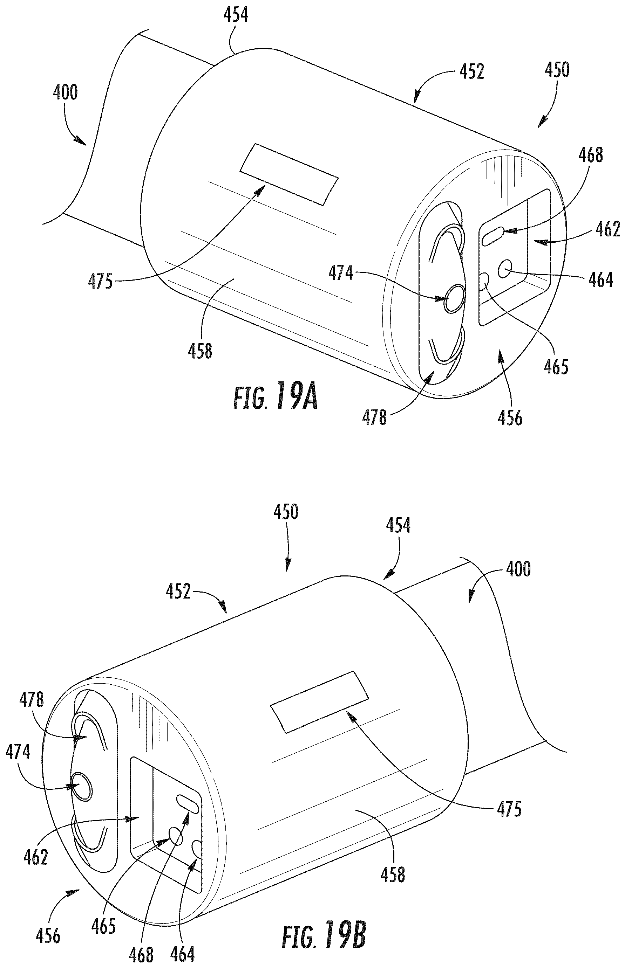

Figs. 19A and 19B illustrate an exemplary embodiment of a coupler device 450. The coupler device 450 serves as an accessory component for currently existing endoscopes. The device seals and covers infection prone areas of the scope to prevent ingress of debris, fluid, or other unwanted matter that could lead to bacterial contamination and decreased performance of the scope. In addition, the coupler device 450 provides a flexible working channel for instruments to be inserted into the scope. The flexible working channel can be angularly adjustable with ease. As shown, in the preferred embodiments, the coupler device 450 may be used with an end viewing scope 400 or other end viewing scope instrument. It is understood, of course, that the coupler device 450 may be adapted for use with side-viewing scopes as well, such as a duodenoscope. In addition, the coupler device 450 can be used with all types of scopes for different medical applications. The end viewing scope 400 shown here is merely for illustrative purposes.

As FIGS. 19A and 19B illustrate, the coupler device 450 may comprise a main body 452 with a proximal end 454 and a distal end 456 and an outer surface 458 surrounding main body 452. The proximal end 454 attaches onto a working end of an end viewing scope 400, extending the working end portion of the scope 400. The distal end 456 may include an optical lens 465 and light guide 464 and a scope washer opening 468, which is used to push fluid across the scope camera to wash debris off the camera and is also used to push air across the camera to dry the camera and insufflate the patient's gastrointestinal tract. Distal end 456 may further include an open area 462 substantially aligned with lens 465 and light guide 464 and scope washer opening 468 to facilitate forward or end viewing of the surgical site and to allow egress of fluid from scope washer opening 468 into the surgical site (and/or egress of air that may be passed over light guide 464 to dry the camera or that may be passed into the surgical site to insufflate a portion of the site).

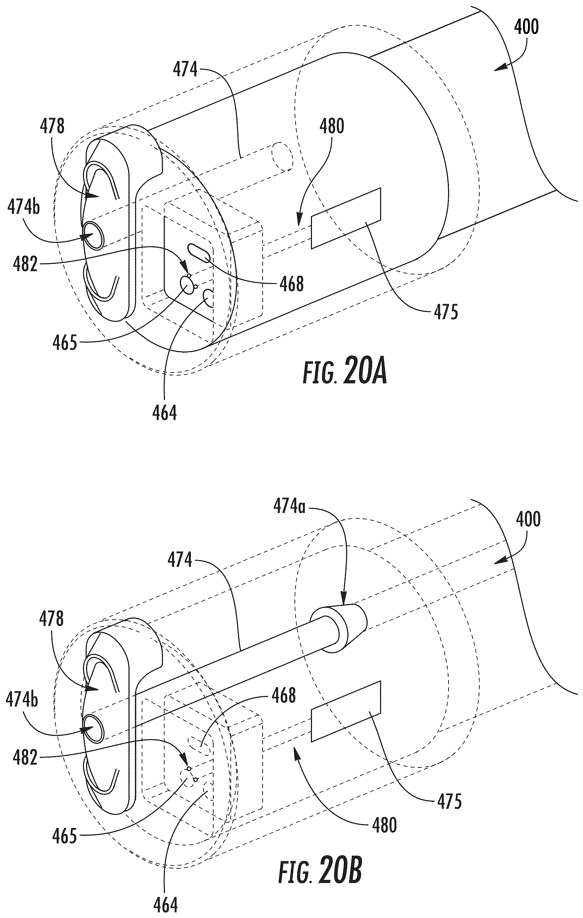

As shown in Figs. 29A and 20B, coupler device 450 further comprises a flexible working channel extension 474 having a proximal end 474a configured for attachment to a working channel of scope 400 and an open distal end 474b. Open distal end 474b of working channel extension 474 is surrounded by a flexible membrane 478. This flexible membrane 478 serves as a protective hood or covering for the working end of the coupler device 450, providing for flexible articulation while sealing out debris, fluid, bacteria or other unwanted matter.

Coupler device 450 comprises one or more receivers 475 on, or within, the outer surface of main body 452. Receivers 475 are preferably configured to receive energy and/or electrical signals from a remote source, as discussed above. As shown in FIG. 20B, coupler device 450 may include actuator (not shown) within main body 452 configured to adjust the focus of optical lens 465. The actuator may include, for example, a controller, a power supply, such as a battery or other suitable source of power, and one or more coupling elements for coupling either directly to optical lens 465 or indirectly through another element within the endoscope. The power supply provides energy to the optical lens 465 through the coupling elements in order to adjust the surface profile or curvature of the lens, thereby adjusting the focus of the lens.

As shown in Figs. 20A and 20B, receiver(s) 475 are coupled to one or more coupling elements 480. Coupling elements 480 are positioned such that, when proximal end 454 of coupler device 450 is attached to the working end of an endoscope, coupling elements 480 are in operational proximity to optical lens 465. Operational proximity as defined herein means that the coupling elements 480 are positioned so as to apply energy to optical lens to alter the curvature of the lens. Coupling elements 480 may be in direct contact with lens, adjacent to lens, or in direct contact or adjacent to another coupling element or actuator in the endoscope. In the latter configuration, coupling elements 480 transmit energy to the other coupling element or actuator, which then functions to transmit the energy to lens 465 and/or to an actuator that alters the curvature of lens 465.

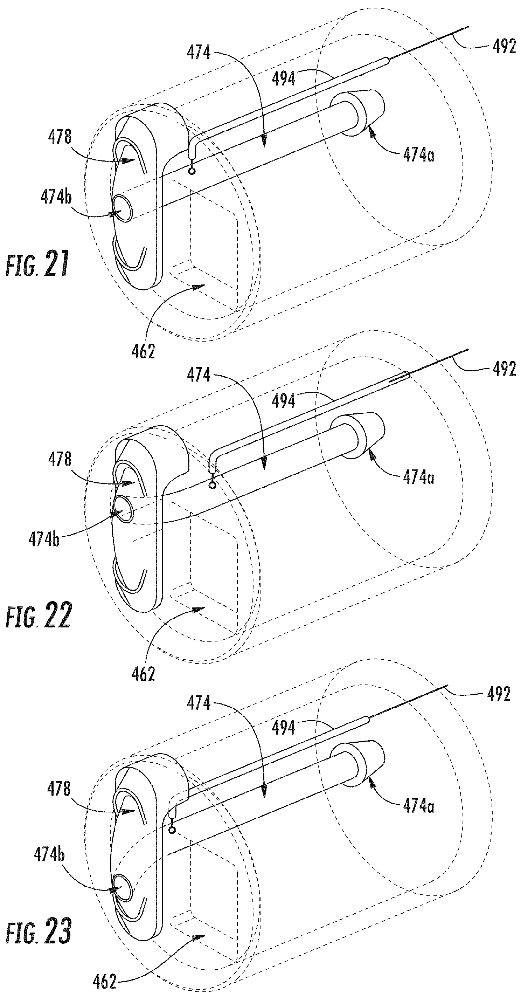

As shown in FIGS. 20A and 20B, the end viewing scope 400 may comprise a light guide 464, lens 465 and washer opening 468. The coupler device 450 cooperates with each of these components of the scope 400 to provide a fully functioning scope. The coupler device 450 does not interfere with the scope's ability to emit a clear image, but instead reduces the risk of contamination with each use. This benefit is achieved by providing a coupler device 450 which attaches to the working end components of the scope 400, and seals around the working end. The coupler device 450 provides an extension of the scope's working channel. The working channel extension 474 of the coupler device 450 in FIG. 20 is flexible and may contact the scope's working channel by a sealed connection, as shown in FIG. 22B, at the proximal end 474a of the working channel extension. The distal end 474b of the working channel extension 474 serves as an exit portal for instruments to pass through the scope 400 to reach different areas of the body.

Additionally, the coupler device 400 provides a further seal around the cable 492 of the scope 400 (see FIG. 21). Because the coupler device 450 seals the cable 492, risk of debris influx, fluids, bacteria and other matter build up behind the elevator and working channel is reduced significantly. This influx of debris, bacteria and other matter is believed to be the reason for drug resistant infections with current scopes today. While preventing influx, the coupler device 450 advantageously maintains flexibility to move the working channel extension 474.

In use, the scope's working channel extension 474 permits passage of instruments down the scope working channel 490 and through and out the working channel extension 474 of the device 450 for assessment and treatment of tissue and other matter. Such instruments may include cannula, catheters, stents and stent delivery systems, papillotomes, wires, other imaging devices including mini-scopes, baskets, snares and other devices for use with a scope in a lumen. This working channel extension 474 is flexible enough that the cable 492 of the scope 400 can raise and lower the working channel extension 474 so that instruments can be advanced down and out of the working channel extension distal end (or exit portal) 474b of the coupler 450 at various angles that are transverse to the longitudinal axis of scope 400.

Referring now to FIGS. 21-23, coupler device 450 comprises a disposable external sheath 494 that can receive an interior actuating cable 492 of the scope. This cable 492 can be attached to the flexible working channel extension 474 of the coupler device 450. As described below, actuation of the cable effects movement of the working channel extension 474. The external sheath 494 may be configured to attach directly to the scope 400, such as by winding around the outside of the scope or by a friction fit connection. In embodiments, multiple cables may be included in one or more sheaths to provide for articulation in other quadrants than the single axis articulation with elevators in current duodenoscopes.

As FIGS. 21-23 illustrate, in use when the cable 492 of the scope 400 is actuated, the flexible working channel extension 474 of the coupler device moves or adjusts to this actuation, either upwards or downwards such that the instruments exiting working channel extension 474 exit at an angle transverse to the longitudinal axis of scope 400. In FIG. 21, cable 492 is relaxed and allows working channel extension 474 to extend substantially parallel to the longitudinal axis of scope 400. In FIG. 22, the cable 492 has been withdrawn proximally such that the working channel extension 474 is raised and distal end 474b of working channel extension 474 is directed upwards. In this orientation, an instrument will exit working channel extension 474 in a direction slightly upward (relative to the drawing) from the longitudinal axis of scope 400. FIG. 23 illustrates cable 492 being advanced in the distal direction to translate working channel extension 474 downward (relative to the drawing) such that an instrument extends downward from distal end 474b.

Of course, it will be recognized that other configurations are possible. For example, the scope 400 may have two cables 492, with each cable located on either side of working channel extension 474. In this embodiment, proximal translation of the upper cable, for example, will cause working channel extension 474 to be oriented upwards or above the longitudinal axis of scope 400. Likewise, proximal translation of the lower cable will cause working channel extension 474 to be oriented downwards or below the longitudinal axis of scope 400. In other embodiments, the scope cables may be configured to pull working channel extension 474 laterally from side to side (relative to the drawing). In this manner, working channel extension 474 may be oriented at any angle 360 degrees around the longitudinal axis of scope 400. In some embodiments, the cable(s) may be attached directly to the working channel extension or to other devices that can be articulated and cause the working channel extension to change its angle of exit. In some embodiments, the articulation ability of the coupler device may be created with an actuator embedded in the coupler device, which is disposable and therefore thrown away after the procedure. A more complete description of alternative coupler devices can be found in commonly assigned, co-pending U.S. Application Serial No. 16/717,804, filed December 17, 2019, the complete disclosure of which is hereby incorporated herein by reference for all purposes as if copied and pasted herein.

Hereby, all issued patents, published patent applications, and non-patent publications that are mentioned in this specification are herein incorporated by reference in their entirety for all purposes, to the same extent as if each individual issued patent, published patent application, or non-patent publication were specifically and individually indicated to be incorporated by reference.

Other embodiments will be apparent to those skilled in the art from consideration of the specification and practice of the embodiment disclosed herein. It is intended that the specification and examples be considered as exemplary only, with a true scope and spirit of the embodiment being indicated by the following claims.

The invention may further be described without limitation and by way of example only by the following embodiments:
1. A device for use with an endoscope having an optical lens, the device comprising:
   a main body comprising a visualization section for allowing viewing of tissue by the endoscope and a proximal end configured for removable attachment to a distal end portion of the endoscope; and
   an actuator within the main body positioned to be in operational proximity to the optical lens when the proximal end of the main body is attached to the distal end portion of the endoscope, the actuator being configured to alter a characteristic of the optical lens.
2. The device of clause 1, wherein the actuator is configured to alter a focal length of the optical lens
3. The device of clause 2, wherein the actuator is configured to alter a curvature of the optical lens.
4. The device of clause 1, wherein the actuator comprises a receiver configured to wirelessly receive energy from a source of energy and to transmit the energy to the optical lens.
5. The device of clause 1, wherein the actuator comprises a power supply and a coupling element coupled to the power supply, wherein the coupling element is configured to transfer energy from the power supply to the optical lens when the proximal end of the main body is attached to the distal end portion of the endoscope
6. The device of clause 3, wherein the optical lens comprises a fluid, the actuator being configured to alter a shape of the fluid to alter the curvature of the optical lens.
7. The device of clause 6, wherein the actuator is configured to change a pressure of the fluid.
8. The device of claim 6, wherein the actuator is configured to change a volume of the fluid within the lens.
9. The device of clause 6, wherein the actuator is configured to supply electrical energy to the fluid.
10. The device of clause 9, wherein the actuator further comprises one or more electrodes coupled to the receiver and configured to contact the optical lens when the proximal end is attached to the distal end portion of the endoscope.
11. The device of clause 10, wherein the receiver is configured to convert the energy to an electrical current and to apply the electric current across the electrodes.
12. The device of clause 4, wherein the energy comprises an electromagnetic field.
13. The device of clause 12, wherein the receiver comprises a magnetic coil.
14. The device of clause 12, wherein the receiver comprises an electrode.
15. The device of clause 4, wherein the energy comprises electromagnetic radiation.
16. The device of clause 15, wherein the receiver comprises an antenna.
17. An endoscope system comprising:
   an endoscope comprising an elongate shaft having a proximal end and a distal end, and an optical lens disposed at the distal end of the shaft; and
   a coupler device comprising:
      a main body comprising a visualization section for allowing viewing of tissue by the endoscope and a proximal end configured for removable attachment to the distal end of the endoscope; and
      an actuator within the main body operatively coupled to the optical lens and configured to alter a characteristic of the optical lens.
18. The device of clause 17, wherein the actuator is configured to alter a focal length of the optical lens
19. The device of clause 18, wherein the actuator is configured to alter a curvature of the optical lens.
20. The device of clause 17, wherein the actuator comprises a receiver configured to wirelessly receive energy from a source of energy and to transmit the energy to the optical lens.
21. The device of clause 17, wherein the actuator comprises a power supply and a coupling element coupled to the power supply, wherein the coupling element is configured to transfer energy from the power supply to the optical lens when the proximal end of the main body is attached to the distal end portion of the endoscope
22. The device of clause 19, wherein the optical lens comprises a fluid, the actuator being configured to alter a shape of the fluid to alter the curvature of the optical lens.
23. The device of clause 22, wherein the actuator is configured to change a pressure of the fluid.
24. The device of clause 22, wherein the actuator is configured to change a volume of the fluid within the lens
25. The device of clause 22, wherein the actuator is configured to supply electrical energy to the fluid.
26. The device of clause 25, wherein the actuator further comprises one or more electrodes coupled to the receiver and configured to contact the optical lens when the proximal end is attached to the distal end portion of the endoscope.
27. The device of clause 26, wherein the receiver is configured to convert the energy to an electrical current and to apply the electric current across the electrodes.
28. The device of clause 17, wherein the energy comprises an electromagnetic field.
29. The device of clause 28, wherein the receiver comprises a magnetic coil.
30. The device of clause 28, wherein the receiver comprises an electrode.
31. The device of clause 17, wherein the energy comprises electromagnetic radiation.
32. The device of clause 31, wherein the receiver comprises an antenna.
33. The endoscope of clause 37, wherein the actuator further comprises one or more electrodes coupled to the receiver and wherein the receiver is configured to convert the energy to an electrical current and to apply the electric current across the electrodes.
34. The endoscope of clause 38, wherein the energy comprises an electromagnetic field.
35. The endoscope of clause 38, wherein the energy comprises electromagnetic radiation.
36. A device for use with an endoscope having an optical lens and a working channel, the device comprising:
   a main body comprising a visualization section for allowing viewing of tissue by the endoscope and a proximal end configured for removable attachment to a distal end portion of the endoscope;
   an actuator within the main body positioned to be in operational proximity to the optical lens when the proximal end of the main body is attached to the distal end portion of the endoscope, the actuator being configured to alter a characteristic of the optical lens; and
   wherein the actuator is configured to articulate an instrument passing through the working channel of the endoscope.
37. The device of clause 36, wherein the actuator comprises a receiver configured to wirelessly receive energy from a source of energy.
38. The device of clause 36, wherein the actuator comprises a power supply and one or more coupling elements coupled to the power supply, wherein the coupling elements are positioned to be in operational proximity to the optical lens when the proximal end of the main body is attached to the distal end portion of the endoscope.
39. The device of clause 36, further comprising a flexible working channel extension within the main body, wherein the flexible working channel has an open distal end and a proximal end configured to couple with a distal end of the working channel of the endoscope.
40. The device of clause 39, wherein the actuator is configured to articulate the flexible working channel extension to change an angle of the open distal end relative to the coupler device.

## Claims

1. An endoscope (100) comprising:
an elongate shaft (114, 151) having a proximal end and a distal end, and an optical lens (160; 46) disposed at the distal end of the shaft; and
a coupler device (10) comprising a main body (12) comprising a visualization section for allowing viewing of tissue by the endoscope and an actuator (79) within the main body operatively coupled to the optical lens and configured to alter a characteristic of the optical lens.

2. The endoscope of claim 1, wherein the coupler device is arranged on the distal end of the shaft.

3. The endoscope of claim 1 or 2, wherein the main body comprises a proximal end (14) configured for removable attachment to the distal end of the endoscope.

4. The endoscope of any one of the preceding claims, wherein the actuator is configured to alter a focal length of the optical lens

5. The endoscope of any one of the preceding claims, wherein the actuator is configured to alter a curvature of the optical lens.

6. The endoscope of any one of the preceding claims, wherein the actuator comprises a receiver (75) configured to wirelessly receive energy from a source of energy and to transmit the energy to the optical lens.

7. The endoscope of any one of claims 1 through 5, wherein the actuator comprises a power supply and a coupling element (77) coupled to the power supply, wherein the coupling element is configured to transfer energy from the power supply to the optical lens when the proximal end of the main body is attached to the distal end portion of the endoscope

8. The endoscope of any one of the preceding claims, wherein the optical lens comprises a fluid (175, 176), the actuator being configured to alter a shape of the fluid to alter the curvature of the optical lens.

9. The endoscope of claim 8, wherein the actuator is configured to change a pressure of the fluid; and/or
wherein the actuator is configured to change a volume of the fluid within the lens; and/or
wherein the actuator is configured to supply electrical energy to the fluid.

10. The endoscope of any one of claims 1 through 3, wherein the actuator further comprises one or more electrodes (177, 178) coupled to a receiver and configured to contact the optical lens when the proximal end is attached to the distal end portion of the endoscope.

11. The endoscope of claim 10, wherein the receiver is configured to convert the energy to an electrical current and to apply the electric current across the electrodes.

12. The endoscope of claim 10 or 11, wherein the receiver comprises an antenna (304).

13. The endoscope of claim 11, wherein the energy comprises an electromagnetic field.

14. The endoscope of claim 11, wherein the energy comprises electromagnetic radiation.

15. The endoscope of any one of claims 10 through 14, wherein the receiver comprises a magnetic coil (328).
